# EUROPEAN PATENT APPLICATION

(11) **EP 3 750 497 A2**
(43) Date of publication of application: **16.12.2020**
(21) Application number: 20175773.9
(22) Date of filing: 20.05.2020
(51) Int. Cl.: A61B 17/80, A61B 17/82

(54) **STERNAL CLOSURE STRAP DEVICE**

(30) Priority: 21.05.2019 US 201962850613 P
(71) Applicant: Jace Medical, LLC, Warsaw, IN 46580 (US)
(72) Inventor: Detweiler, Jason F., Warsaw, in 46582 (US); Steffensmeier, Scott, Winona Lake, IN 46590 (US)
(74) Representative: Herzog IP Patentanwalts GmbH

(57) **Abstract**

A bone closure strap device (10) that includes a securing member (14) that has a body with at least one strap receiving hole (20) and at least one locking feature (24) movably connected to the body. The at least one locking feature is located adjacent to the at least one strap receiving hole. The bone closure strap device also includes a strap (12) that has a first end fixedly connected to the securing member. The strap is configured for looping around the bone portions, being inserted into the at least one strap receiving hole, and being secured in the at least one strap receiving hole by the at least one locking feature.

## Description

### Cross Reference To Related Applications

This is a non-provisional application based upon U.S. provisional patent application serial no. 62/850613, entitled "STERNAL CLOSURE STRAP DEVICE", filed May 21, 2019, which is incorporated herein by reference.

### BACKGROUND OF THE INVENTION

### 1. Field of the Invention

The present invention relates to bone fixation devices, and, more particularly, to sternal closure strap devices.

### 2. Description of the Related Art

Sternal fixation or closure devices are used to fix together portions of a sternum in a reapproximation medical procedure. Various surgical procedures require that the sternum is cut in half and separated before the desired surgical procedure(s) is conducted in the thoracic cavity. In so doing, the surgeon will conduct a partial or median sternotomy wherein a longitudinal incision is made in the sternum which then allows the portions, e.g. halves, of the sternum to be separated. After the surgical procedure(s) has been conducted, the sternum must be reapproximated using one or more sternal fixation devices to hold and secure the portions of the sternum together. Generally, each sternal fixation device will engage or otherwise wrap around the sternal portions in order to hold and secure the sternal portions together.

A typical sternal fixation device comprises a stainless-steel wire. In operation, the wire is wrapped around the sternal portions or weaved within through-holes in each sternal portion to form a loop that engages with both of the sternal portions. Thereafter, the ends of the wire are twisted around one another to tighten the loop and secure the sternal portions together. Such wire-type sternal fixation devices are cost-effective. However, these wire-type sternal fixation devices may dig or cut into the sternum, which damages the sternum and diminishes the integrity of the sternal bond. In severe cases, the damage to the sternum may lead to the partial or complete separation of the sternal portions. Also, given the twist-tie nature of the wires, these wire-type sternal fixation devices may insufficiently or unevenly compress the sternal halves together, which may undesirably result in movement, e.g. rubbing, of the sternal portions and/or surrounding tissue.

What is needed in the art is an easy-to-use bone fixation device that evenly secures bone portions together.

### SUMMARY OF THE INVENTION

The present invention provides sternal closure devices for closing and retaining halves of a sternum. The sternal closure devices are strap-type or cable-tie type devices that generally include a securing member and a strap connectable to the securing member. The strap loops around and retains the halves of the sternum.

The invention in one form is directed to a bone closure strap device for securing bone portions. The bone closure strap device includes a securing member with a body that has at least one strap receiving hole and at least one locking feature movably connected to the body. The at least one locking feature is located adjacent to the at least one strap receiving hole. The bone closure strap device further includes a strap including a first end fixedly connected to the securing member. The strap is configured for looping around the bone portions. The strap is further configured for being inserted into the at least one strap receiving hole. The strap is further configured for being secured in the at least one strap receiving hole by the at least one locking feature.

The invention in another form is directed to a bone closure strap device for securing bone portions. The bone closure strap device includes a securing plate with a body that has at least one strap receiving hole, a plurality of fastener receiving holes each configured for receiving a respective fastener, and at least one locking feature located adjacent to the at least one strap receiving hole. The bone closure strap device further includes at least one strap connectable to the securing plate. The at least one strap is configured for looping around the bone portions. The at least one strap is further configured for being inserted into the at least one strap receiving hole. The at least one strap is further configured for being secured in the at least one strap receiving hole by the at least one locking feature.

The invention in another form is directed to a bone closure strap device for securing bone portions. The bone closure strap device includes a two-part securing member with a first portion and a second portion. The first portion is removably connected to the second portion. The second portion includes at least one strap receiving hole. The bone closure strap device further includes a strap configured for looping around the bone portions. The strap is further configured for being inserted into the at least one strap receiving hole. The strap is further configured for being secured in the at least one strap receiving hole by the first portion.

An advantage of the present invention is that the bone closure device is both cost-effective and efficient because the strap alone may dually attach the securing member to juxtaposed bone portions and securely hold the bone portions together.

Another advantage of the present invention is that the bone closure device may be efficiently connected to juxtaposed bone portions because each fastener may dually attach the securing member to the bone portions and secure the strap within the securing member.

Yet another advantage of the present invention is that the bone closure device may be easily attached to juxtaposed bone portions by simply looping the strap around the bone portions, inserting the strap through the securing member, tightening the strap, and manipulating the locking feature or inserting the fastener to secure the strap within the securing member.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
FIG. 1 is an exploded view of an embodiment of a bone closure strap device with a securing member and a strap;
FIG. 2 is a perspective view of the bone closure strap device of FIG. 1;
FIG. 3 is a perspective view of another embodiment of a bone closure strap device with a securing member that has an integrated locking feature and a strap;
FIG. 4 is a perspective view of the bone closure strap device of FIG. 3;
FIG. 5 is a perspective view of another embodiment of a bone closure strap device with a securing plate and multiple straps;
FIG. 6 is a top view of the securing plate of the bone closure strap device of FIG. 5;
FIG. 7 is a perspective view of another embodiment of a bone closure strap device with a securing plate and a single strap;
FIG. 8 is another perspective view of the bone closure strap device of FIG. 7;
FIG. 9 is a perspective view of another embodiment of a bone closure strap device with a securing member with two locking features and a strap;
FIG. 10 is a perspective view of the securing member of the bone closure strap device of FIG. 9;
FIG. 11 is an exploded view of another embodiment of a bone closure device with a two-part securing member and a strap;
FIG. 12 is an exploded view of the securing member of the bone closure device of FIG. 11;
FIG. 13 is a perspective view of another embodiment of a bone closure device with a two-part locking feature and a strap;
FIG. 14 is another perspective view of the bone closure device of FIG. 13; and
FIG. 15 is an exploded view of the locking feature of the bone closure device of FIGS. 13-14.

Corresponding reference characters indicate corresponding parts throughout the several views. The exemplifications set out herein illustrate embodiments of the invention and such exemplifications are not to be construed as limiting the scope of the invention in any manner.

### DETAILED DESCRIPTION OF THE INVENTION

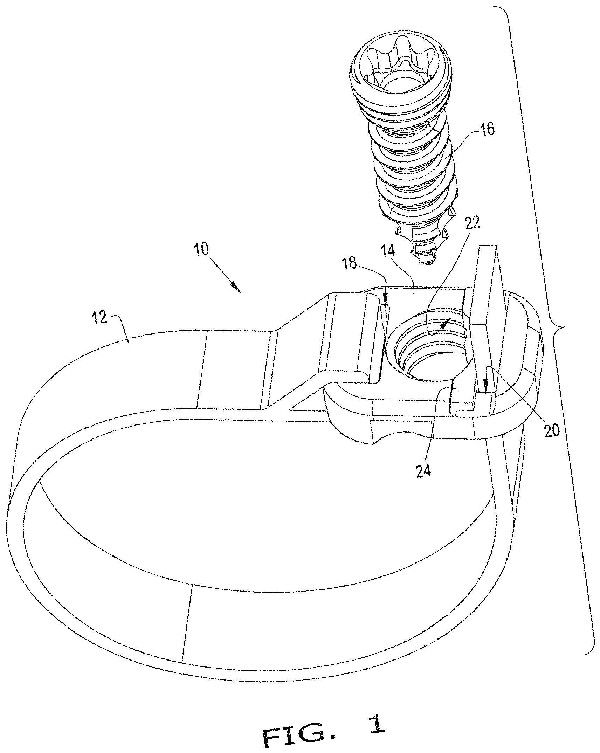

Referring now to the drawings, and more particularly to FIGS. 1-2, there is shown an embodiment of a sternal closure strap device 10 for closing and retaining halves of a sternum of a patient after undergoing a surgical procedure. The strap device 10 may generally include a strap 12, a securing member 14, and a fastener 16. The strap device 10 may function as a cable-tie type device to loop around and retain the juxtaposed halves of the sternum. For example, a user may wrap the strap 12 in between a pair of intercostal spaces and around the halves of the sternum, feed the strap 12 through the securing member 14, tighten or shorten the thus created loop, and insert the fastener 16 to thereby dually retain the strap 12 and attach the securing member 14 to one or both halves of the sternum.

The strap 12 may be fixedly and/or removably attached to the securing member 14. For example, the strap 12 may be fixedly attached to the securing member 14 at one end, via a looped connection, and may be removably attached to the securing member 14 at the other end, via the fastener 16. In other words, the free end of the strap 12 may be inserted into and through the securing member 14. The strap 12 may be looped around any desired portions of the sternum. The strap 12 may be in the form of any desired strap 12 and may be composed of any desired material, such as a fibrous material, metal, and/or plastic. The strap 12 may have a uniform and rectangular cross-section. However, the strap 12 may have any desired shape. For instance, the strap 12 may have a varying contour with different widths.

The securing member 14 may be removably connected to the sternum of a patient. The securing member 14 may have a first end with a first receiving hole 18 for fixedly mounting the strap 12, a second end with a second receiving hole 20 for slidably receiving the strap 12, a middle section with a third receiving hole 22 for receiving the fastener 16, and a locking feature 24 for selectively abutting and securing the strap 12 after the strap 12 has been tightened around the bone portions. The first and second receiving holes 18, 20 which receive the strap 12 may extend through the body of the securing member 14. The first and second receiving holes 18, 20 may have a rectangular cross-section; however, they may have any desired cross-sectional shape. The second, strap receiving hole 20 allows the strap 12 to be inserted therethrough. The third receiving hole 22 which receives the fastener 16 may be substantially in the middle of the securing member 14. The third receiving hole 22 may be threaded. The securing member 14 may be composed of any desired material, such as metal and/or plastic.

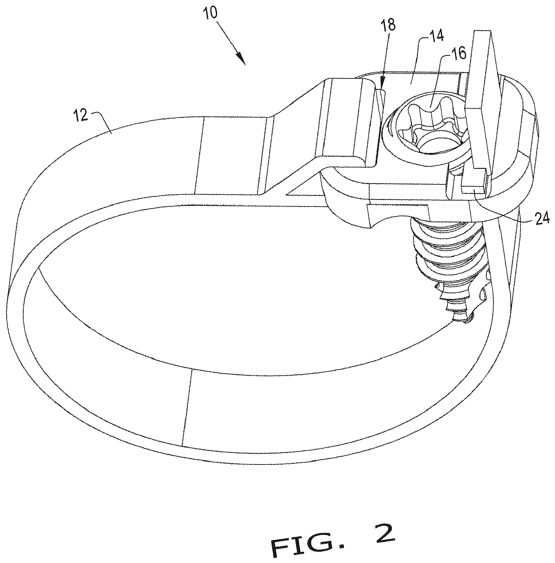

The locking feature 24 is associated with the third receiving hole 22, the second receiving hole 20, and the strap 12. The locking feature 24 is located adjacent to the second, strap receiving hole 20 and the third, fastener receiving hole 22. The locking feature 24 may slide relative to the body of the securing member 14 such that the locking feature 24 slides to at least partially overlap with or extend over the third receiving hole 22 in an unlocked position (FIG. 1) or the second receiving hole 20 in a locked position to secure the strap 12 (FIG. 2). In this regard, as the fastener 16 is inserted into the third receiving hole 22, the fastener 16 contacts and axially slides, e.g. pushes, the locking feature 24 against the strap 12 to thereby secure the strap 12 against the distal wall of the second receiving hole 20 (FIG. 2). The locking feature 24 may be in the form of a locking bar 24, which may be substantially rectangular with a flat side that selectively engages with the strap 12 and a contoured side, which may be threaded, that selectively engages with the fastener 16. The locking feature 24 may axially slide within a grooved profile of the body of the securing member 14 (unnumbered). The locking feature 24 may be the only locking feature of the securing member 14. The locking feature 24 may be composed of any desired material, such as metal and/or plastic.

The fastener 16 may be selectively inserted into the securing member 14 for dually screwing into the sternum of the patient, thus attaching the securing member 14 to the sternum, and moving the locking feature 24 for securing the strap 12 within the securing member 14. The fastener 16 may be in the form of a screw. For example, the fastener 16 may be an orthopedic bone screw. However, the fastener 16 may be in the form of any desired fastener.

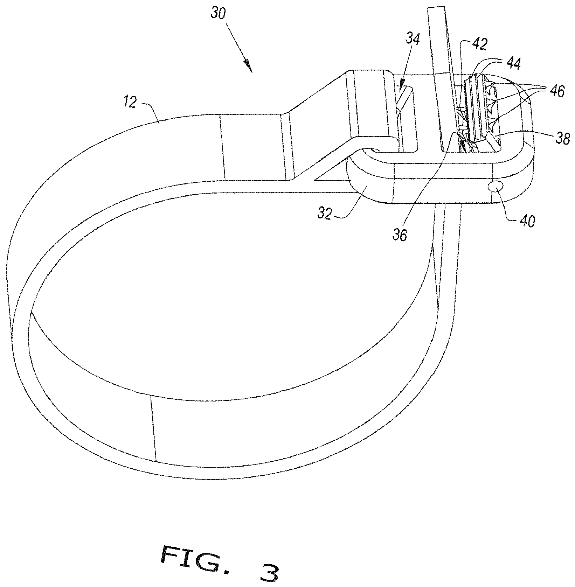

Referring now to FIGS. 3-4, there is shown another embodiment of a sternal closure strap device 30 for closing and retaining halves of a sternum of a patient after undergoing a surgical procedure. The strap device 30 may generally include a strap 12 and a securing member 32. Similarly to the strap device 10, the strap device 30 may also function as a cable-tie type device to loop around and retain the juxtaposed halves of the sternum.

The securing member 32 may contact and secure the halves of the sternum. The securing member 32 may have a first end with a first receiving hole 34 for fixedly mounting the strap 12, a second end with a second receiving hole 36 for slidably receiving the strap 12, and a pivoting locking feature 38 for selectively abutting and securing the strap 12. The securing member 32 may be composed of any desired material, such as metal and/or plastic. It should be appreciated that securing member 32 may or may not be removably attached to the sternum of the patient by way of a fastener and/or adhesive. For instance, the securing member 32 may not include a fastener and may thus be secured to the sternum only by way of the strap 12.

The first and second receiving holes 34, 36, which receive the strap 12, may extend through the body of the securing member 32. The second, strap receiving hole 36 allows the free end of the strap 12 to be inserted therethrough. The first and second receiving holes 34, 36 may have a rectangular cross-section; however, they may have any desired cross-sectional shape.

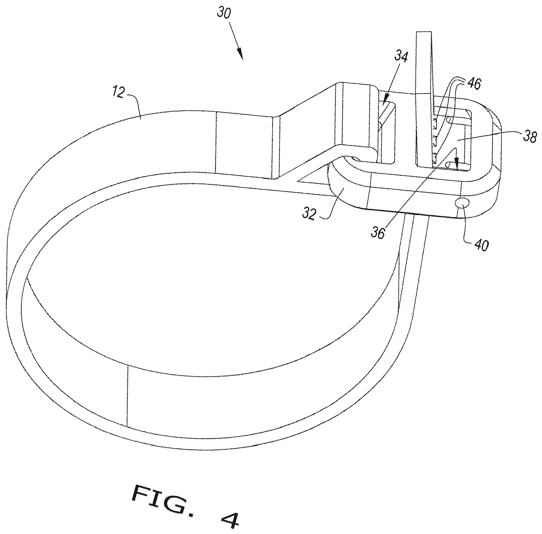

The locking feature 38 is associated with the second receiving hole 36 and the strap 12. The locking feature 38 is located adjacent to the second, strap receiving hole 36. The locking feature 38 may be pivotally connected to the body of the securing member 32 at pivot 40. The locking feature 38 may upwardly pivot away from the second receiving hole 36 into an unlocked position for threading the strap 12 through the second receiving hole 36 (FIG. 3). The locking feature 38 may downwardly pivot into a locked position to secure the strap 12 (FIG. 4). For instance, a user may push the locking feature 38 downwardly so that the locking feature 38 contacts and applies a locking force onto the strap 12; and thereby, the strap 12 is secured in between the body of the locking feature 38 and one or more surfaces of the second receiving hole 36. The locking feature 38 may include at least one lower protrusion 42, side protrusion 44, and/or upper protrusion 46. The locking feature 38 may have any desired shape. For example, the locking feature 38 may be an oblong member, such as a locking cam 38, which is pivotally connected to the securing member 32 at one end via pivot 40. The locking feature 38 may be the only locking feature of the securing member 32. The locking feature 38 may be composed of any desired material, such as metal and/or plastic.

The at least one lower protrusion 42 may be in the form of one or more teeth 42 which are equidistantly spaced across the width of the locking feature 38. The lower teeth 42 may have a curved profile, for example a finned profile, which allows the strap 12 to be threaded through the second receiving hole 36 without being obstructed by the lower teeth 42. The lower teeth 42 may allow the strap 12 to pass through the second receiving hole 36 in only one direction, i.e., upwardly, and if the strap 12 is pulled in the opposite direction, i.e., downwardly, the lower teeth 42 will engage the strap 12 and further close the locking feature 38, thereby preventing slippage of the strap 12. The at least one middle protrusion 44 may be in the form of one or more groves and/or ridges 44. The side ridges 44 may be uniform members which extend across the width of the side of the locking feature 38. The side ridges 44 may contact and secure the strap 12 in the locked position (FIG. 4). The at least one upper protrusion 46 may be in the form of one or more teeth 46. The upper teeth 46 may have a curved profile, which may curve in an opposing manner to the lower teeth 42. The upper teeth 46 may engage the strap 12 when the locking feature 38 is fully closed in the locked position. If the locking feature 38 experiences a lateral distraction force, the upper teeth 46 will ensure the locking feature 38 is tightened, thus generating additional clamping pressure to prevent slippage of the strap 12. The at least one protrusion 42, 44, 46 of the locking feature 38 may be composed of any desired material, such as metal and/or plastic.

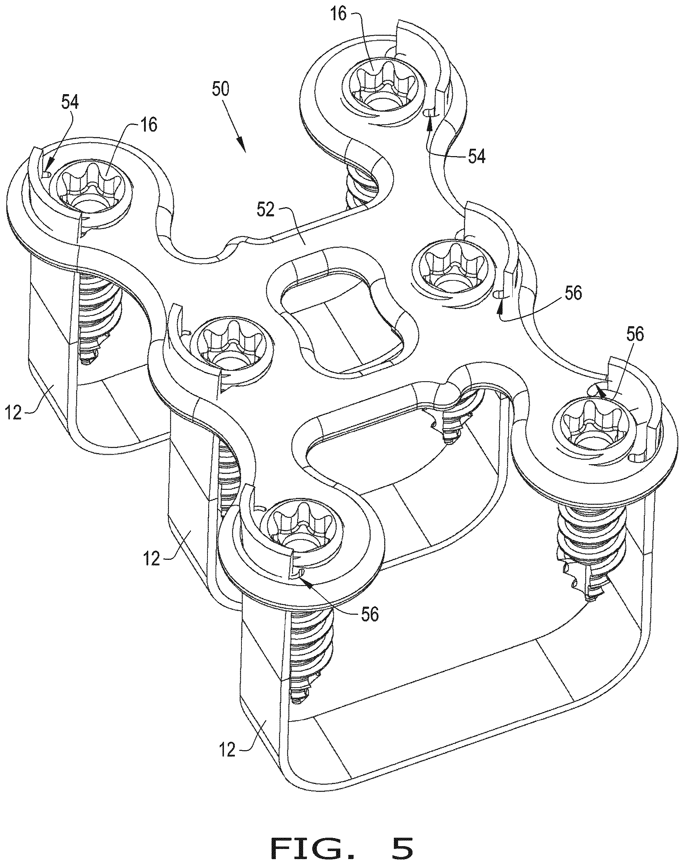

Referring now to FIGS. 5-6, there is shown another embodiment of a sternal closure strap device 50. The strap device 50 may generally include multiple straps 12, a securing member 52 in the form of a securing plate 52, and multiple fasteners 16. The strap device 50 may function as a cable-tie type device to loop around and retain the juxtaposed halves of the sternum.

The securing plate 52 may contact and secure the halves of the sternum. The body of the securing plate 52 may have receiving holes 54, 56 for slidably receiving the straps 12, multiple fastener receiving holes 58 for receiving a respective fastener 16, and multiple locking features 60, 62 each being associated with a respective fastener receiving hole 58 and movable for selectively securing the straps 12 within the securing plate 52 (FIG. 5). For example, the securing plate 52 may have one or more two-part, i.e., split, locking features 60, with respective strap receiving holes 54, and/or one or more single body locking features 62, with respective strap receiving holes 56. The securing plate 52 may have any desired shape. For example, the securing plate 52 may have an "H"-shape. The securing plate 52 may be composed of any desired material, such as metal and/or plastic.

The strap receiving holes 54, 56, which receive the straps 12, may extend through the body of the securing plate 52. Each strap receiving hole 54, 56 allows the strap 12 to be inserted therethrough and tightened before a respective fastener 16 is inserted into the respective fastener receiving hole 58 for securing the looped and tightened strap 12. The strap receiving holes 54, 56 may have a crescent cross-section; however, they may have any desired cross-sectional shape. The fastener receiving holes 58 may be threaded in order to receive the fasteners 16.

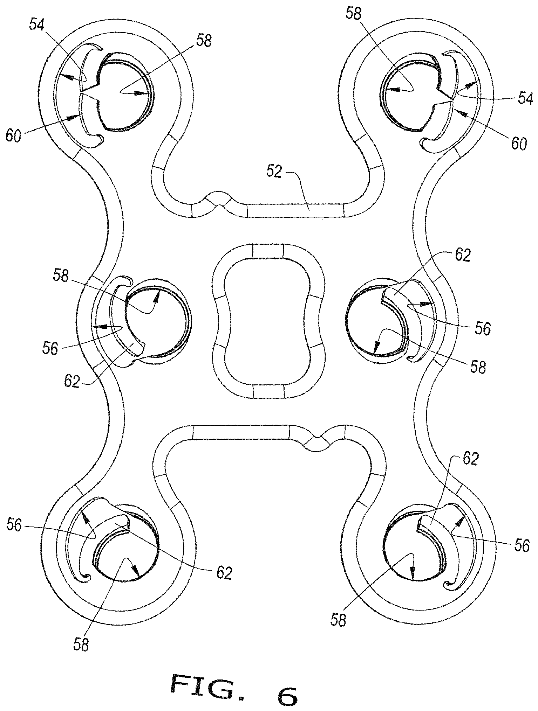

Each locking feature 60, 62 is located adjacent to its respective strap receiving hole 54, 56. Therein, each locking feature 60, 62 and strap receiving hole 54, 56 is associated with a respective fastener receiving hole 58. Each locking feature 60, 62 may be integrally formed with or connected to the body of the securing plate 52. As shown, each locking feature 60, 62 is integrally or monolithically formed as part of the body of the securing plate 52. Each locking feature 60, 62 may be movable relative to the surrounding body of the securing plate 52. Hence, the locking features may move, e.g. bend or flex, to at least partially extend into and out of the respective receiving holes 54, 56, 58. Each locking feature 60, 62 may be inwardly biased, axially into its respective fastener receiving hole 58, in an unlocked position (FIG. 6). Thereby, each locking feature 60, 62 may be pushed outwardly to abut against the straps 12 in a locked position by way of the fasteners 16 applying a force onto the locking features 60, 62 as the fasteners 16 are inserted into their receiving holes 58 (FIG. 5). The inside of the locking features 60, 62 may be threaded similarly to the fastener receiving holes 58, and the outside of the locking features 60, 62 may include abrasions and/or protrusions. Each locking feature 60, 62 may have any desired shape. Each locking feature 60, 62 may be composed of any desired material, such as metal and/or plastic.

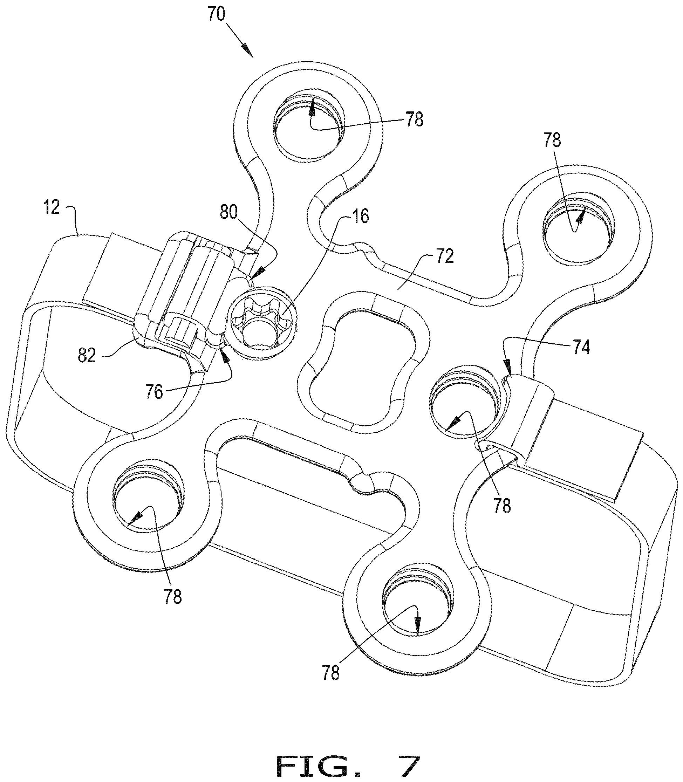

Referring now to FIGS. 7-8, there is shown another embodiment of a sternal closure strap device 70. The strap device 70 may generally include a single strap 12, a securing member 72 in the form of a securing plate 72, and multiple fasteners 16 (only one is shown in FIG. 7). The strap device 70 may function as a cable-tie type device to loop around and retain the juxtaposed halves of the sternum.

The securing plate 72 may contact and secure the halves of the sternum. The securing plate 72 may have a first strap receiving hole 74 for fixedly attaching the strap 12, a second strap receiving hole 76 for slidably receiving the strap 12, multiple fastener receiving holes 78 for receiving a respective fastener 16, and a locking feature 80 for selectively securing the strap 12 within the securing plate 72. For example, the securing plate 72 may only have a single locking feature 80 associated with only one strap receiving hole 76 and respective fastener receiving hole 78. The securing plate 72 may also have a strap fastener 82, for example a buckle, for allowing the strap 12 to be looped therearound. The securing plate 72 may have any desired shape. For example, the securing plate 72 may have an "H"-shape. The securing plate 72 may be composed of any desired material, such as metal and/or plastic.

The first and second strap receiving holes 74, 76 which receive the strap 12 may extend through the body of the securing plate 72. The second, strap receiving hole 74 allows the free end of the strap 12 to be inserted therethrough. The first and second strap receiving holes 74, 76 may have a crescent cross-section; however, they may have any desired cross-sectional shape. The fastener receiving holes 78 may be threaded in order to receive the fasteners 16.

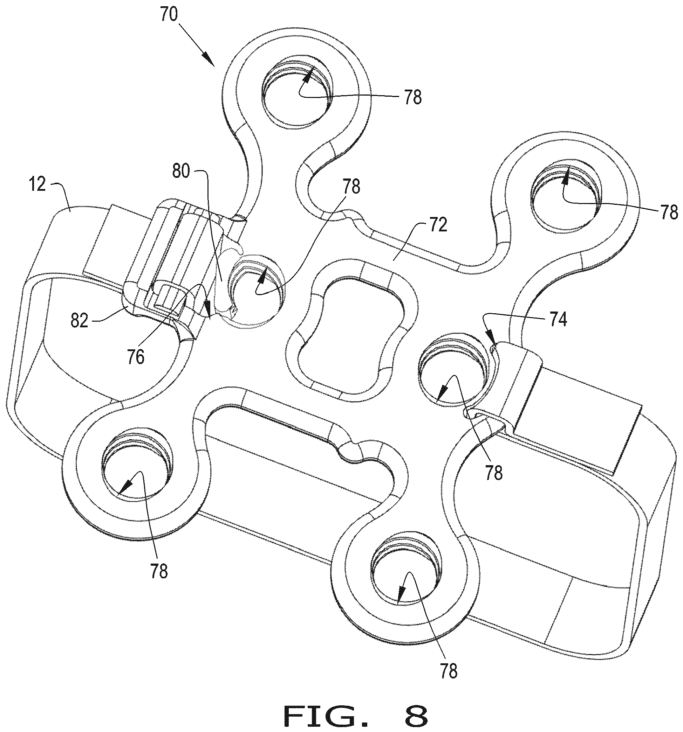

The locking feature 80 may be substantially similar to the locking feature 62 as discussed above. The locking feature 80 is located adjacent to the strap receiving hole 76. The locking feature 80 may move in between an unlocked position (FIG. 8) and a locked position upon inserting the fastener 16 to secure the strap 12 (FIG. 7). The locking feature 80 may have any desired shape and may be composed of any desired material, such as metal and/or plastic.

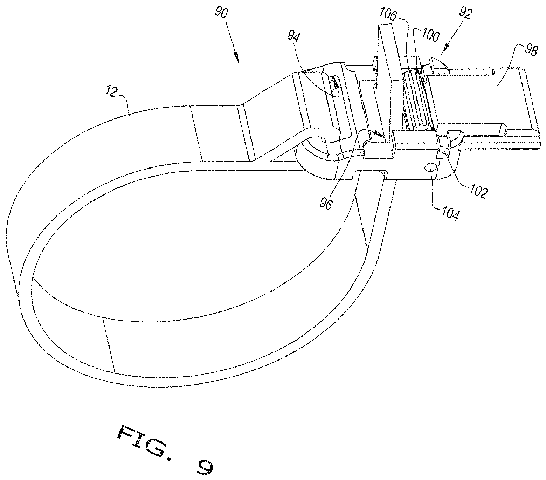

Referring now to FIGS. 9-10, there is shown another embodiment of a sternal closure strap device 90. The strap device 90 may generally include a strap 12 and a securing member 92. The strap device 90 may function as a cable-tie type device to loop around and retain the juxtaposed halves of the sternum.

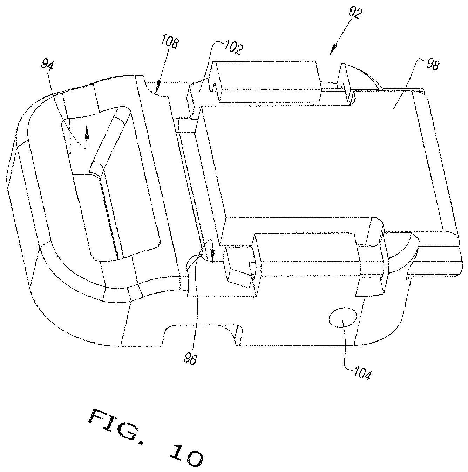

The securing member 92 may contact and secure the halves of the sternum. The securing member 92 may have a first end with a first receiving hole 94 for fixedly mounting the strap 12, a second end with a second receiving hole 96 for slidably receiving the strap 12, and a first, sliding locking feature 98 and a second, pivoting locking feature 100. The locking features 98, 100 may respectively slide and rotate in between an unlocked position (FIG. 9) and a locked position (FIG. 10) to jointly and selectively secure the strap 12.

The first and second receiving holes 94, 96 which receive the strap 12 may extend through the body of the securing member 92. The second, strap receiving hole 96 allows the free end of the strap 12 to be inserted therethrough. The first and second receiving holes 94, 96 may have a rectangular cross-section; however, they may have any desired cross-sectional shape.

The sliding locking feature 98 is associated with and located adjacent to the strap receiving hole 96. The sliding locking feature 98 may slide within a groove or designated railing within the body of the securing member 92 (unnumbered). The sliding locking feature 98 may be substantially rectangular and may have locking end hooks 102. The locking end hooks 102 may be deformable. Each locking end hook 102 may engage with a portion, e.g. a wall or ledge, of the body of the securing member 92 in the locked position (FIG. 10). The sliding locking features 98 may have any desired shape and may be composed of any desired material, such as metal and/or plastic.

The pivoting locking feature 100 is also associated with and located adjacent to the strap receiving hole 96. The pivoting locking feature 100 may be pivotally connected to the body of the securing member 92 at pivot 104. The pivoting locking feature 100 may be substantially similar to the locking feature 38 as discussed above. Therein, the pivoting locking feature 100 may include one or more protrusions. For instance, the pivoting locking feature 100 may include side protrusions 106. The side protrusions 106 may be in the form of ridges 106. The pivoting locking feature 100 may have any desired shape and may be composed of any desired material, such as metal and/or plastic.

In the unlocked position, the sliding locking feature 98 may be slid away from the second receiving hole 96 and the pivoting locking feature 100 may be rotated upwardly so that neither locking feature 98, 100 may obstruct the second receiving hole 96 (FIG. 9). As the sliding locking feature 98 slides toward the second receiving hole 96, the sliding locking feature 98 may contact and move the pivoting locking feature 100 downwardly to abut up against the strap 12. When the sliding locking feature 98 is slid into the locked position, the pivoting locking feature 98 may be oriented directly toward the strap 12 so that the side protrusions 106 engage with the strap 12 and secure the strap 12 in between the body of the locking feature 100 and one or more surfaces of the second receiving hole 96 (FIG. 10). Furthermore, in the locked position, the sliding locking feature 98 is slid substantially over the second receiving hole 96 such that the sliding locking feature 98 pinches the strap 12 against the body of the securing member 92. Therein, the locking end hooks 102 removably connect to the body of the securing member 92 to hold the sliding locking feature 98 in place.

Furthermore, the securing member 92 may also include a cutting member 108 (FIG. 10). The cutting member 108 may be positioned adjacent to the second receiving hole 96 and may be configured for cutting the strap 12 to a desired length. For example, when the sliding locking feature 98 is slid into its locked position it may push the strap 12 to contact the cutting member 108, which in turn cuts the strap 12. Thereby the strap 12 may be automatically cut by the cutting member 108 upon positioning the sliding locking feature 98 into the locked position. The cutting member 108 may be in the form of a sharp edge 108 of the body of the securing member 92. Alternatively, the cutting member 108 may not be integrally formed with the body of the securing member 92. For example, the cutting member 108 may be a razor fixedly attached to the securing member 92. The cutting member 108 may be composed of any desired material, such as metal and/or plastic.

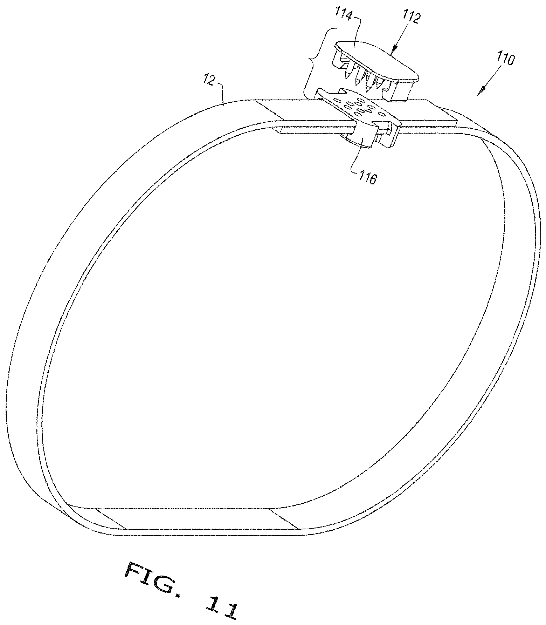

Referring now to FIGS. 11-12, there is shown another embodiment of a sternal closure strap device 110. The strap device 110 may generally include a strap 12 and a securing member 112. The strap device 110 may function as a cable-tie type device to loop around and retain the juxtaposed halves of the sternum. The securing member 112 may be positioned in between an unlocked position (FIGS. 11-12) and a locked position for selectively securing the strap 12.

The securing member 112 may contact and secure the halves of the sternum. The securing member 112 may be in the form of a two-part securing member 112 with an upper, male portion 114 and a lower, female portion 116 which slidably receives the strap 12. The securing member 112 may be composed of any desired material, such as metal and/or plastic.

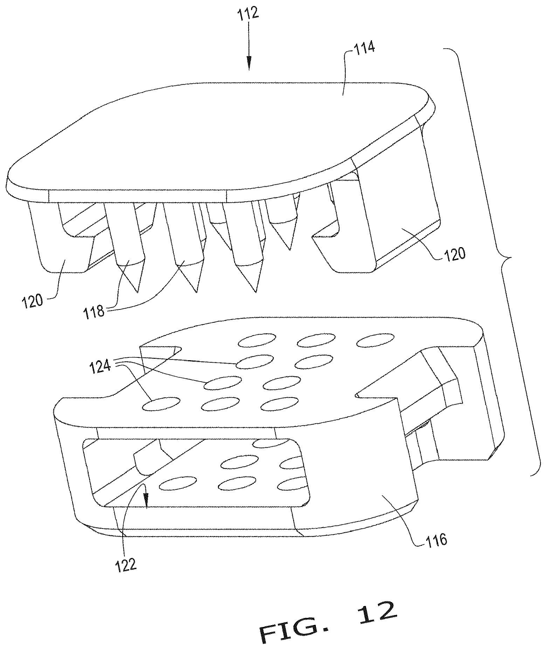

The upper portion 114 may be removably connected to the lower portion 116. The upper portion 114 may include protrusions 118, for example spikes 118, and hooks 120 for removably connecting to the lower portion 116 (FIG. 12). The lower portion 116 may include a strap receiving hole 122 for receiving the strap 12 and multiple receiving holes 124 for receiving the spikes 118 of the upper portion 114 (FIG. 12). In the unlocked position, the upper portion 114 does not obstruct the strap receiving hole 122 of the lower portion 116, and thereby the strap 12 may be threaded through the strap receiving hole 122. In more detail, the strap 12 may be inserted through the strap receiving hole 122 so that the strap 12 overlaps itself inside of the strap receiving hole 122. In the locked position, the upper portion 114 may be engaged with the lower portion 116 so that the spikes 118 extend through the strap receiving hole 122 and pierce and the overlapped strap 12; thus, securing the strap 12 in the locked position.

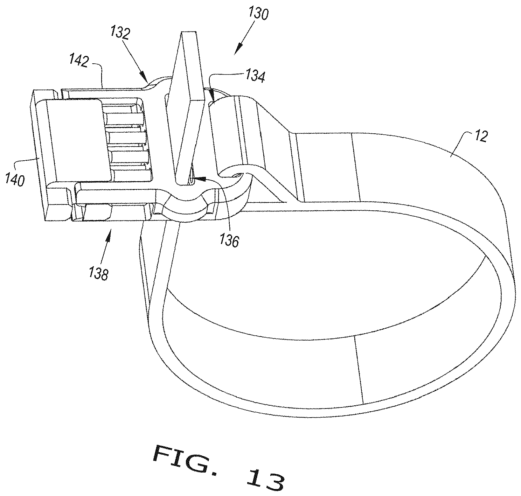

Referring now to FIGS. 13-15, there is shown another embodiment of a sternal closure strap device 130. The strap device 130 may generally include a strap 12 and a securing member 132. The strap device 130 may function as a cable-tie type device to loop around and retain the juxtaposed halves of the sternum.

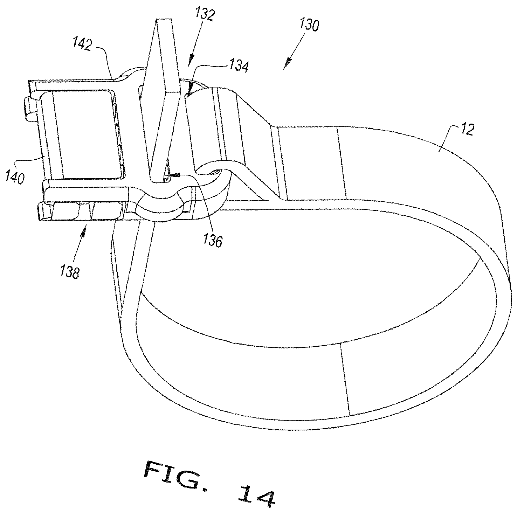

The securing member 132 may be positioned in between an unlocked position (FIG. 13) and a locked position for selectively securing the strap 12 (FIG. 14). The securing member 132 may contact and secure the halves of the sternum. The securing member 132 may generally include a first receiving hole 134 for fixedly mounting the strap 12, a second receiving hole 136 for slidably receiving the strap 12, and a two-part locking feature 138 with a male and female portion 140, 142. The securing member 132 may be composed of any desired material, such as metal and/or plastic.

The receiving holes 134, 136 may extend through the body of the securing member 132. The second, strap receiving hole 136 allows the strap 12 to be inserted therethrough. The receiving holes 134, 136 may have a rectangular cross-section; however, they may have any desired cross-sectional shape.

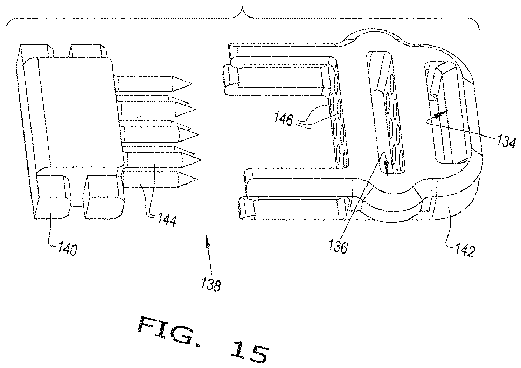

The male portion 140 may be removably connected to the female portion 142. The male portion 140 may also slide relative to the female portion 142 upon being coupled to the female portion 142. The male portion 140 may include protrusions 144, for example spikes 144 (FIG. 15). The female portion 142 includes the strap receiving hole 136 for receiving the free end of the strap 12. The female portion 142 may include receiving holes 146 for receiving the spikes 144 of the male portion 140 (FIG. 15). Hence, in the unlocked position, the spikes 144 do not obstruct the second receiving hole 136 so that the strap 12 may be threaded through the second receiving hole 136 (FIG. 13). In the locked position, the spikes 144 extend through the second receiving hole 136 and the strap 12 to thereby secure the strap 12 (FIG. 15).

The securing member 132 may additionally include retaining features or members that prevent the male and female portions 140, 142 from disconnecting or disassociating from one another. For example, the securing member 132 may include two or more protrusions on the male portion 140 and receiving slots on the female portion 142 which act in conjunction to retain the male portion 140 within the female portion 142 as the male portion 140 slides in between the unlocked and locked positions. In other words, the male portion 140 may move from the unlocked position to the locked position without completely separating from the female protrusion 142. Also, once in the locked position, the protrusions may prevent the male portion 142 from sliding rearwardly away from the female portion 142. In more detail, a leading set of protrusions on the male portion 140 may allow the male portion 140 to slide relative to the female portion 142 whilst retaining the male portion 140 in the female portion 142, and a trailing set of protrusions on the male portion 140, which are spaced apart from the leading set of protrusions, may lock or otherwise prevent the movement of the male portion 140 relative to the female portion 142 in the locked position.

It should be appreciated that any sternal closure strap device 10, 30, 50, 70, 90, 110, 130 described herein may be used to couple any desired bone portions. For example, the sternal closure strap devices 10, 30, 50, 70, 90, 110, 130 may be used to close bone portions of a rib bone.

While this invention has been described with respect to at least one embodiment, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

### Further Embodiments:

|1| A bone closure strap device for securing bone portions, comprising:
   a securing member including a body with at least one strap receiving hole and at least one locking feature movably connected to the body, the at least one locking feature being located adjacent to the at least one strap receiving hole; and
   a strap including a first end being fixedly connected to the securing member, the strap being configured for looping around the bone portions, the strap being further configured for being inserted into the at least one strap receiving hole, the strap being further configured for being secured in the at least one strap receiving hole by the at least one locking feature.
|2| The bone closure strap device of embodiment |1|, wherein the body of the securing member includes two strap receiving holes, one strap receiving hole fixedly connecting the first end of the strap to the securing member and another strap receiving hole configured for receiving the strap upon the strap looping around the bone portions.
|3| The bone closure strap device of embodiment |1| or |2|, wherein the body of the securing member further includes a fastener receiving hole, the fastener receiving hole being configured for receiving a fastener therein.
|4| The bone closure strap device of embodiment |1| to |3|, wherein the at least one locking feature is slidably connected to the body of the securing member such that upon insertion of the fastener in the fastener receiving hole the at least one locking feature slides in between an unlocked position for at least partially extending over the fastener receiving hole and a locked position for at least partially extending over the at least one strap receiving hole to engage with and secure the strap.
|5| The bone closure strap device of embodiment |1| to |4|, wherein the at least one locking feature is pivotally connected to the body of the securing member such that the at least one locking feature is pivotable in between an unlocked position for allowing the strap to be inserted through the at least one strap receiving hole and a locked position for at least partially extending over the at least one strap receiving hole to engage with and secure the strap.
|6| The bone closure strap device of embodiment |1| to |5|, wherein the at least one locking feature is in the form of a locking cam.
|7| The bone closure strap device of embodiment |5| to |6|, wherein the at least one locking feature includes at least one of a lower protrusion, a side protrusion, and an upper protrusion for engaging with the strap.
|8| The bone closure strap device of embodiment |1| to |7|, wherein the securing member includes at least two locking features engageable with the strap, the at least two locking features including a sliding locking feature slidably connected to the body of the securing member and a pivoting locking feature pivotally connected to the body of the securing member.
|9| The bone closure strap device of embodiment |8|, wherein the sliding locking feature is located adjacent to and engageable with the pivoting locking feature, the sliding locking feature is configured for sliding in between an unlocked position and a locked position, the sliding locking feature is further configured for contacting and pivoting the pivoting locking feature such that pivoting locking feature engages with and secures the strap in the locked position.
|10| The bone closure strap device of embodiment |1| to |9|, wherein the securing member further includes a cutting member located adjacent to the at least one strap receiving hole, the cutting member is configured for cutting the strap.
|11| A bone closure strap device for securing bone portions, comprising:
   a securing plate including a body with at least one strap receiving hole, a plurality of fastener receiving holes each being configured for receiving a respective fastener, and at least one locking feature located adjacent to the at least one strap receiving hole; and
   at least one strap connectable to the securing plate, the at least one strap being configured for looping around the bone portions, the at least one strap being further configured for being inserted into the at least one strap receiving hole, the at least one strap being further configured for being secured in the at least one strap receiving hole by the at least one locking feature.
|12| The bone closure strap device of embodiment 1111, wherein the at least one locking feature is integrally formed with the body, the at least one locking feature is configured for flexing to secure the at least one strap.
|13| The bone closure strap device of embodiment |11| or |12|, wherein the body of the securing plate includes a plurality of strap receiving holes, each strap receiving hole of the plurality of strap receiving holes being associated with a receptive fastener receiving hole of the plurality of fastener receiving holes, wherein the at least one locking feature includes a plurality of locking features, wherein the at least one strap includes a plurality of straps, each strap of the plurality of straps being insertable into a respective pair of strap receiving holes of the plurality of strap receiving holes.
|14| The bone closure strap device of embodiment |13|, wherein each locking feature of the plurality of locking features is inwardly biased into a respective fastener receiving hole of the plurality of fastener receiving holes such that the respective fastener upon being inserted into the respective fastener receiving hole moves each locking feature of the plurality of locking features outwardly to engage with and secure each strap of the plurality of straps.
|15| The bone closure strap device of embodiment |11| to |14|, wherein the at least one strap receiving hole includes only one strap receiving hole which is associated with one fastener receiving hole of the plurality of fastener receiving holes, wherein the at least one locking feature includes only one locking feature, wherein the at least one strap includes only one strap having a first end fixedly connected to the securing plate.
|16| A bone closure strap device for securing bone portions, comprising:
   a two-part securing member with a first portion and a second portion, the first portion is removably connected to the second portion, the second portion includes at least one strap receiving hole; and
   a strap configured for looping around the bone portions, the strap being further configured for being inserted into the at least one strap receiving hole, the strap being further configured for being secured in the at least one strap receiving hole by the first portion.
|17| The bone closure strap device of embodiment |16|, wherein the first portion includes a plurality of spikes configured for piercing and extending through the strap to secure the strap in the at least one strap receiving hole.
|18| The bone closure strap device of embodiment |16| or |17|, wherein the first portion further includes a pair of hooks for removably connecting to the second portion.
|19| The bone closure strap device of embodiment |16| to |17|, wherein the second portion includes only one strap receiving hole configured for receiving the strap, wherein the strap is configured for overlapping itself within the strap receiving hole.
|20| The bone closure strap device of embodiment |16| to |19|, wherein the strap includes a first end, wherein the second portion includes two strap receiving holes, one strap receiving hole fixedly connecting the first end of the strap to the second portion and another strap receiving hole configured for receiving the strap upon the strap looping around the bone portions.

## Claims

1. A bone closure strap device for securing bone portions, comprising:
a securing member including a body with at least one strap receiving hole and at least one locking feature movably connected to the body, the at least one locking feature being located adjacent to the at least one strap receiving hole; and
a strap including a first end being fixedly connected to the securing member, the strap being configured for looping around the bone portions, the strap being further configured for being inserted into the at least one strap receiving hole, the strap being further configured for being secured in the at least one strap receiving hole by the at least one locking feature.

2. The bone closure strap device of claim 1, wherein the body of the securing member includes two strap receiving holes, one strap receiving hole fixedly connecting the first end of the strap to the securing member and another strap receiving hole configured for receiving the strap upon the strap looping around the bone portions.

3. The bone closure strap device of claim 1, wherein the body of the securing member further includes a fastener receiving hole, the fastener receiving hole being configured for receiving a fastener therein.

4. The bone closure strap device of claim 3, wherein the at least one locking feature is slidably connected to the body of the securing member such that upon insertion of the fastener in the fastener receiving hole the at least one locking feature slides in between an unlocked position for at least partially extending over the fastener receiving hole and a locked position for at least partially extending over the at least one strap receiving hole to engage with and secure the strap.

5. The bone closure strap device of claim 1, wherein the at least one locking feature is pivotally connected to the body of the securing member such that the at least one locking feature is pivotable in between an unlocked position for allowing the strap to be inserted through the at least one strap receiving hole and a locked position for at least partially extending over the at least one strap receiving hole to engage with and secure the strap.

6. The bone closure strap device of claim 5, wherein the at least one locking feature is in the form of a locking cam.

7. The bone closure strap device of claim 5, wherein the at least one locking feature includes at least one of a lower protrusion, a side protrusion, and an upper protrusion for engaging with the strap.

8. The bone closure strap device of claim 1, wherein the securing member includes at least two locking features engageable with the strap, the at least two locking features including a sliding locking feature slidably connected to the body of the securing member and a pivoting locking feature pivotally connected to the body of the securing member.

9. The bone closure strap device of claim 8, wherein the sliding locking feature is located adjacent to and engageable with the pivoting locking feature, the sliding locking feature is configured for sliding in between an unlocked position and a locked position, the sliding locking feature is further configured for contacting and pivoting the pivoting locking feature such that pivoting locking feature engages with and secures the strap in the locked position.

10. The bone closure strap device of claim 1, wherein the securing member further includes a cutting member located adjacent to the at least one strap receiving hole, the cutting member is configured for cutting the strap.

11. A bone closure strap device for securing bone portions, comprising:
a securing plate including a body with at least one strap receiving hole, a plurality of fastener receiving holes each being configured for receiving a respective fastener, and at least one locking feature located adjacent to the at least one strap receiving hole; and
at least one strap connectable to the securing plate, the at least one strap being configured for looping around the bone portions, the at least one strap being further configured for being inserted into the at least one strap receiving hole, the at least one strap being further configured for being secured in the at least one strap receiving hole by the at least one locking feature.

12. The bone closure strap device of claim 11, wherein the at least one locking feature is integrally formed with the body, the at least one locking feature is configured for flexing to secure the at least one strap.

13. The bone closure strap device of claim 11, wherein the body of the securing plate includes a plurality of strap receiving holes, each strap receiving hole of the plurality of strap receiving holes being associated with a receptive fastener receiving hole of the plurality of fastener receiving holes, wherein the at least one locking feature includes a plurality of locking features, wherein the at least one strap includes a plurality of straps, each strap of the plurality of straps being insertable into a respective pair of strap receiving holes of the plurality of strap receiving holes.

14. The bone closure strap device of claim 13, wherein each locking feature of the plurality of locking features is inwardly biased into a respective fastener receiving hole of the plurality of fastener receiving holes such that the respective fastener upon being inserted into the respective fastener receiving hole moves each locking feature of the plurality of locking features outwardly to engage with and secure each strap of the plurality of straps.

15. The bone closure strap device of claim 11, wherein the at least one strap receiving hole includes only one strap receiving hole which is associated with one fastener receiving hole of the plurality of fastener receiving holes, wherein the at least one locking feature includes only one locking feature, wherein the at least one strap includes only one strap having a first end fixedly connected to the securing plate.

16. A bone closure strap device for securing bone portions, comprising:
a two-part securing member with a first portion and a second portion, the first portion is removably connected to the second portion, the second portion includes at least one strap receiving hole; and
a strap configured for looping around the bone portions, the strap being further configured for being inserted into the at least one strap receiving hole, the strap being further configured for being secured in the at least one strap receiving hole by the first portion.

17. The bone closure strap device of claim 16, wherein the first portion includes a plurality of spikes configured for piercing and extending through the strap to secure the strap in the at least one strap receiving hole.

18. The bone closure strap device of claim 17, wherein the first portion further includes a pair of hooks for removably connecting to the second portion.

19. The bone closure strap device of claim 17, wherein the second portion includes only one strap receiving hole configured for receiving the strap, wherein the strap is configured for overlapping itself within the strap receiving hole.

20. The bone closure strap device of claim 16, wherein the strap includes a first end, wherein the second portion includes two strap receiving holes, one strap receiving hole fixedly connecting the first end of the strap to the second portion and another strap receiving hole configured for receiving the strap upon the strap looping around the bone portions.
